# EUROPEAN PATENT SPECIFICATION

(11) **EP 1 646 391 B1**
(45) Date of publication and mention of the grant of the patent: **15.11.2006**
(21) Application number: 04740899.2
(22) Date of filing: 08.07.2004
(51) Int. Cl.: A61K 31/5365, A61P 25/02

(54) **USE OF 2H-[1,3]-OXAZINO[3,2-A]INDOLE DERIVATIVES FOR THE TREATMENT OF NEUROPATHIC PAIN**
VERWENDUNG VON 2H-[1,3]-OXAZINO[3,2-A]INDOL-DERIVATEN ZUR BEHANDLUNG VON NEUROPATHISCHEM SCHMERZ
UTILISATION DE DERIVES DE 2H-[1,3]-IXAZINO[3,2-A]INDOLE POUR LE TRAITEMENT DE LA DOULEUR NEUROPATHIQUE

(30) Priority: 18.07.2003 IT MI20031467
(43) Date of publication of application: 19.04.2006
(73) Proprietor: AZIENDE CHIMICHE RIUNITE ANGELINI FRANCESCO A.C.R.A.F. S.p.A., 00181 Roma (IT)
(72) Inventor: GUGLIELMOTTI, Angelo, I-00149 Roma (IT); POLENZANI, Lorenzo, I-00046 Grottaferrata (IT); ALISI, Alessandra, I-00199 Roma (IT); CAZZOLLA, Nicola, I-00041 Albano Laziale (IT)
(74) Representative: Marchi, Massimo
(86) International application number: PCT/EP2004/007633
(87) International publication number: WO 2005/014001

(56) References cited:
- EP-A- 0 884 319
- EP-B- 0 630 376
- WO-A-93/06108
- US-A- 5 817 676
- HOUGHTON L.A. ET AL: "5-HT4 receptor antagonism in irritable bowel syndrome: Effect of SB - 207266 -A on rectal sensitivity and small bowel transit." ALIMENTARY PHARMACOLOGY AND THERAPEUTICS, 13/11 (1437-1444). REFS: 32 ISSN: 0269-2813 CODEN: APTHEN, 1999, XP008037091
- DOAK G.J.; SAWYNOK J.: "Formalin-induced nociceptive behavior and edema: involvement of multiple peripheral 5-hydroxytryptamine receptor subtypes" NEUROSCIENCE, vol. 80, no. 3, 1997, pages 939-949, XP000886556
- DONAHUE ET AL.: "Electrolytic lesion of the anterior cingulate cortex decreases inflammatory, but not neuropathic nociceptive behavior in rats" BRAIN RESEARCH, vol. 897, no. 1-2, 2001, pages 131-138, XP002279567
- BEERS M. AND BERKOW R.: "The Merck Manual of Diagnosis and Therapy, Seventeenth Edition" 1999, MERCK RESEARCH LABORATORIES , WHITEHOUSE STATION, N.J. , XP002279568 page 1371, column 2, paragraph 2 - page 1372, column 2, paragraph 2
- WEI H ET AL: "Influence of two serotonin receptor antagonists in neuropathic rats" PFLUEGERS ARCHIV EUROPEAN JOURNAL OF PHYSIOLOGY, vol. 443, no. Supplement 1, March 2002 (2002-03), page S181, XP001202624 & 81ST ANNUAL JOINT MEETING OF THE PHYSIOLOGICAL SOCIETY, THE SCANDINAVIAN PHYSIOLOGICAL SOCIETY AND T; TUEBINGEN, GERMANY; MARCH 15-19, 2002 ISSN: 0031-6768

## Description

The present invention relates to the use of an indole compound according to claim 1 for the preparation of a pharmaceutical composition for the treatment of neuropathic pain.

European patent application EP-A-0 630 376 relates to a large number of compounds of formula I: 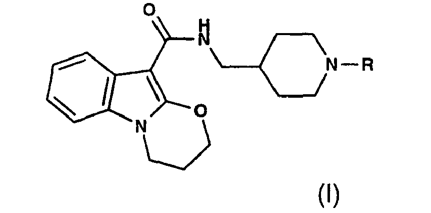 including those wherein
R is H, a linear or branched alkyl chain having from 1 to 12 carbon atoms or an arylalkyl radical.

According to the aforesaid document the compound of formula (I) is active in the treatment or the prophylaxis of gastrointestinal, cardiac and central nervous system disorders.

Hereinafter, the compounds of formula (I) wherein R has the aforesaid meanings or a cyclohexyl group will for brevity be referred to as "Compound (I)".

It has now surprisingly been found that Compound (I) is particularly active in neuropathic pain.

It is known that on average about 10-20% of the adult population suffer from chronic pain. The chronic pain is generally associated with clinical conditions characterised by chronic and/or degenerative lesions.

Typical examples of pathological conditions characterised by chronic pain are rheumatoid arthritis, osteoarthritis, fibromyalgia and neuropathy [Ashburn M A, Staats P S, Management of chronic pain. Lancet 1999; 353: 1865-69].

Chronic pain, in particular neuropathic pain, is often debilitating and is a cause of loss of working capacity and poor quality of life. Consequently, it also results in economic and social losses.

The analgesic drugs currently used in the treatment of neuropathic pain include non-steroidal anti-inflammatories (NSAIDs), antidepressants, opioid analgesics, and anticonvulsants [Woolf C J, Mannion R J. Neuropathic pain: aetiology, symptoms, mechanism, and management. Lancet 1999; 353: 1959-1964].

However, chronic pain and, in particular, neuropathic pain is notoriously difficult to treat with the drugs currently available. Consequently, the development of novel analgesics has always been one of the major targets of the pharmaceutical industry. Moreover, in spite of the many research efforts intended to identify a suitable analgesic compound, there are a significant number of patients for whose pain condition there is still no satisfactory treatment [Scholz J, Woolf C J. Can we conquer pain? Nat Neusci. 2002; 5: 1062-76].

Houghton et al. disclose in Aliment. Pharmacol. Ther. 1999, 13, 1437-1444 that 5-HT₄ receptor antagonists suppress nociceptor response to colorectal distension in rats. SB-207266 A has an effect on rectal sensitivity.

### Brief description of the drawings:

Figure 1 shows the effect of the hydrochloride salt of the compound of formula (I) wherein R = n-butyl on allodynia induced by ligature of the sciatic nerve in the rat.
Figure 2 shows the effect of the hydrochloride salt of the compound of formula (I) wherein R = n-butyl on mechanical hyperalgesia in rats with diabetes induced by streptozotocin.

It is an object of the present invention to use a compound of formula (I), wherein R is H, a linear or branched alkyl chain having from 1 to 12 carbon atoms or an arylalkyl group and of the acid addition salts thereof with pharmaceutically acceptable organic or inorganic acids, to prepare a pharmaceutical composition active in the treatment of neuropathic pain.

Preferably, in the arylalkyl group the alkyl moiety has from 1 to 4 carbon atoms and the aryl moiety is a phenyl or naphthyl ring.

Typical examples of pharmaceutically acceptable organic or inorganic acids are: oxalic, maleic, methanesulphonic, paratoluenesulphonic, succinic, citric, tartaric, lactic, hydrochloric, phosphoric and sulphuric.

Typical examples of pathological conditions characterised by neuropathic pain are diabetes, cancer, immunodeficiency, traumas, ischaemia, multiple sclerosis, sciatic neuralgia, trigeminal neuralgia and post-herpetic syndromes.

Preferably, the pharmaceutical compositions of the present invention are prepared in the form of suitable dosage forms containing an effective dose of at least one Compound (I) or of an acid addition salt thereof with a pharmaceutically acceptable organic or inorganic acid and at least one pharmaceutically acceptable inert ingredient.

Examples of suitable dosage forms are tablets, capsules, coated tablets, granules, solutions and syrups for oral administration; medicated plasters, solutions, pastes, creams and ointments for transdermal administration; suppositories for rectal administration and sterile solutions for administration by the injection or aerosol routes.

Other suitable dosage forms are the sustained release dosage forms and the dosage forms based on liposomes for oral or injection administration.

The dosage forms may also comprise other conventional ingredients such as: preservatives, stabilisers, surfactants, buffers, salts to regulate the osmotic pressure, emulsifiers, sweeteners, colorants and flavourings.

If required by particular therapies, the pharmaceutical composition of the present invention may comprise other pharmacologically active ingredients whose concomitant administration is useful.

The amount of Compound (I) or of an acid addition salt thereof with a pharmaceutically acceptable organic or inorganic acid in the pharmaceutical composition of the present invention can vary over a wide range depending on known factors such as, for example, the type of pathology with which the neuropathic pain to be treated is associated, the severity of the disease, the patient's body weight, the dosage form, the chosen administration route, the number of administrations per day and the efficacy of the chosen compound of formula (I). However, the optimal amount can be determined in a simple and routine manner by the person skilled in the art.

Typically, the amount of Compound (I) or of an acid addition salt thereof with a pharmaceutically acceptable organic or inorganic acid in the pharmaceutical composition of the present invention will be such as to ensure an administration level of from 0.001 to 100 mg/kg/day of Compound (I), as a base. Preferably, the administration level will be of from 0.05 to 50 mg/kg/ day, and still more preferably of from 0.1 to 10 mg/kg/day.

The dosage forms of the pharmaceutical composition of the present invention can be prepared by techniques well known to the pharmaceutical chemist which include mixing, granulating, compressing, dissolving, sterilizing and the like.

The analgesic activity of Compound (I) has been proved by means of two experimental models in the rat: allodynia induced by ligature of the sciatic nerve and mechanical hyperalgesia in diabetic neuropathy induced by streptozotocin.

As is known to the person skilled in the art, the aforesaid experimental models can be considered to be predictive of activity in man.

The experimental model of ligature of the sciatic nerve in the rat is a neuropathy which reproduces a series of responses similar to those observed in man in many traumatic and pathological conditions associated with neuropathic pain. Ligature of the sciatic nerve is in fact capable of inducing a syndrome associated with the activation of specific circuits responsible for the control of the perception of pain and
characterised by the appearance of allodynia, hyperalgesia and spontaneous pain. As is well known, this model is an effective instrument for the study of drugs for use in the treatment of neuropathic pain in man and, in particular, in the control of conditions such as allodynia and hyperalgesia.

In its turn, the diabetic neuropathy induced by streptozotocin in the rat is an insulin-dependent syndrome characterised by a concomitant decrease in the conduction speed of the motor and sensory nerves and the appearance of a series of anomalies in the perception of pain. As is well known, this model is a useful instrument for the study of drugs for use in the treatment of neuropathic pain in man. In particular, the model is a valid example of a large group of neuropathic pain types characterised by phenomena such as hyperalgesia and allodynia due to primary lesions or dysfunctions of the nervous system.

Typical examples of human pathologies characterised by the dysfunctions described in the two experimental models cited above and
characterised by the presence of neuropathic pain are diabetes, cancer, immunodeficiency, trauma, ischaemia, multiple sclerosis, sciatic neuralgia, trigeminal neuralgia and post-herpetic syndromes.

### TESTS

### 1. Allodynia induced by ligature of the sciatic nerve in the rat

Male CD rates of weight 200-250 g on arrival were used.

The allodynia was induced by ligature under anaesthesia of the sciatic nerve of the left hind paw [Seltzer Z, Dubner R, Shir Y. A novel behavioral model of neuropathic pain disorders produced in rats by partial sciatic nerve injury. Pain 1990; 43: 205-218; Bennett G J, Xie Y K. A peripheral mononeuropathy in rat that produces disorders of pain sensation like those seen in man. Pain 1998; 33: 87-107]. After at least two weeks following the ligature of the sciatic nerve, rats which showed a reduction of a least 50% in the response threshold recorded before the operation were selected. The pain threshold was measured by means of a von Frey instrument which, by applying a gradual increase in pressure on the plantar zone of the left hind paw of the rat, makes it possible to record the nocifensive response, expressed in grams, corresponding to the moment at which the animal withdraws its paw.

At 30 minutes, 1, 2 and 4 hrs after the treatment, the pain threshold measured in control animals was compared with that measured in animals treated with the compound under test.

The control animals were treated with same vehicle (water) as was used for administration of the compound under test (hydrochloride salt of the compound of formula (I) wherein R is n-butyl, prepared as disclosed in Example 3, Method 2, of EP-A-0 630 376).

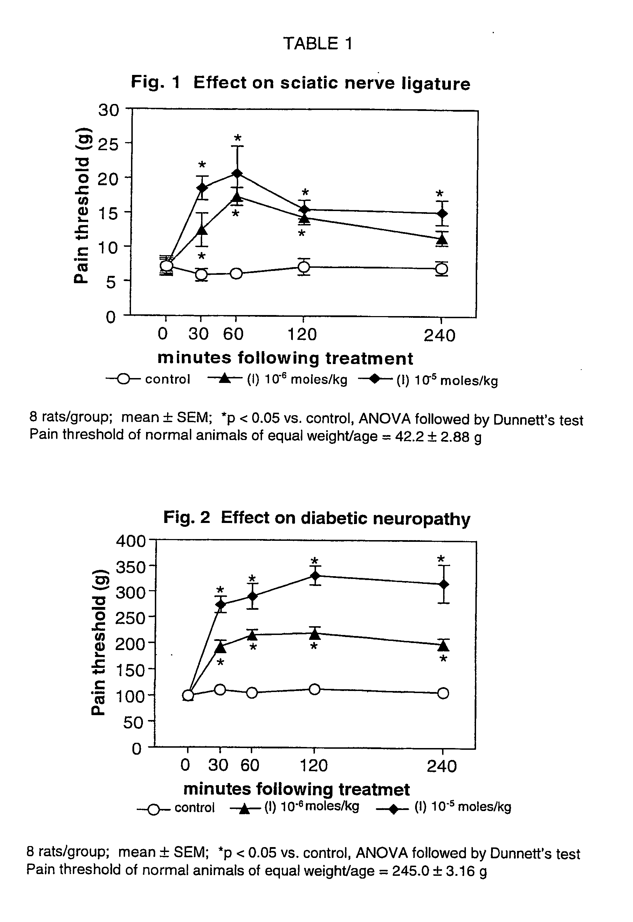

The results are shown in Figure 1.

Similar results were obtained with the hydrochloride salt of the compound of formula (I), wherein R is cyclohexyl, prepared according to Examples 23 of EP-A-0 630 376.

### 2. Mechanical hyperalgesia in rats with diabetes induced by streptozotocin

Male CD rates of weight 240-300 g on arrival were used.

The diabetic syndrome was induced by a single intraperitoneal (i.p.) injection of 80 mg/kg of streptozotocin dissolved in sterile physiological solution [Courteix C, Eschalier A, Lavarenne J. Streptozotocin-induced diabetic rats: behavioural evidence for a model of chronic pain. Pain, 1993; 53: 81-88; Bannon A W, Decker M W, Kim Dj, Campbell J E, Arneric S P. ABT-594, a novel cholinergic channel modulator, is efficacious in nerve ligation and diabetic neuropathy models of neuropathic pain. Brain Res. 1998; 801: 158-63].

After at least three weeks following the injection of streptozotocin, rats with a glycaemia level ≥ 300 mg/dl and a response threshold ≤ 120 g to a mechanical nociceptive stimulus were selected. The glycaemia levels were measured using a reflectometer utilising reactive strips impregnated with glucose oxidase. The pain threshold was measured using an analgesimeter. The instrument, by applying a gradual increase in pressure on the dorsal zone of the left hind paw of the rat, makes it possible to record the nocifensive response, expressed in grams, corresponding to the moment at which the animal withdraws its paw.

At 30 minutes, 1, 2 and 4 hrs after the treatment, the pain threshold measured in control animals was compared with that measured in animals treated with the compound under test.

The control animals were treated with same vehicle (water) as was used for administration of the compound under test (hydrochloride salt of the compound of formula (I) wherein R is n-butyl, prepared as disclosed in Example 3, Method 2, of EP-A-0 630 376).

The results are shown in Figure 2.

Similar results were obtained with the hydrochloride salt of the compound of formula (I), wherein R is cyclohexyl, prepared according to Examples 23 of EP-A-0 630 376.

### Examples

### Example 1

A tablet comprising, as the active principle, the Compound (I) of the present invention, has the following composition:

| | |
|---|---|
| Active principle | 50 mg |
| Lactose monohydrate | 161 mg |
| Dibasic calcium phosphate dihydrate | 161 mg |
| Microcrystalline cellulose | 95 mg |
| Maize starch | 30 mg |
| Sodium carboxymethyl starch | 24 mg |
| Povidone | 11 mg |
| Magnesium stearate | 3 mg |

### Example 2

An ampoule comprising, as the active principle, the Compound (I) of the present invention, has the following composition:

| | |
|---|---|
| Active principle | 25 mg |
| Sorbitol | q.s. for isosmotic solution |
| Water | q.s to 100 ml |

### Example 3

A pharmaceutical composition in granules comprising, as the active principle, a Compound (I) of the present invention, has the following composition:

| | |
|---|---|
| Active principle | 50 mg |
| Maltitol | 1300 mg |
| Mannitol | 2700 mg |
| Saccharose | 1000 mg |
| Citric acid | 20 mg |
| Aspartame | 20 mg |
| Flavourings | 200 mg |

## Claims

1. Use of a compound of formula I: 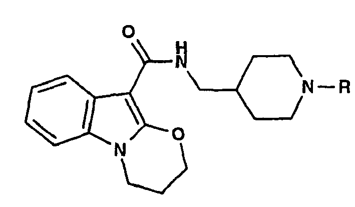 wherein
R is H, a linear or branched alkyl chain having from 1 to 12 carbon atoms, a cyclohexyl or an arylalkyl group,
and of the acid addition salts thereof with pharmaceutically acceptable organic or inorganic acids, for
to prepare a pharmaceutical composition for the treatment of neuropathic pain.

2. Use according to claim 1, **characterized in that** R is an arylalkyl group where the alkyl moiety has from 1 to 4 carbon atoms.

3. Use according to claim 1 or 2, **characterized in that** R is an arylalkyl group where the aryl moiety is a phenyl or naphthyl ring.

4. Use according to claim 1, **characterized in that** R is an n-butyl group.

5. Use according to claim 1, **characterized in that** R is a cyclohexyl group.

## Patentansprüche

1. Verwendung einer Verbindung gemäß Formel I: 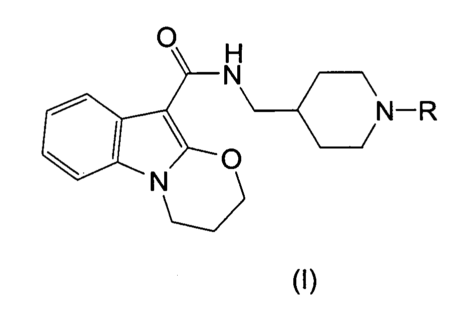 worin
R H, eine lineare oder verzweigte Alkylkette mit ein bis zwölf Kohlenstoffatomen, eine Cyclohexyl- oder Arylalkylgruppe ist,
und von den Säureadditionssalzen davon mit pharmazeutisch annehmbaren organischen oder inorganischen Säuren, zur Herstellung einer pharmazeutischen Zusammensetzung für die Behandlung von neuropathischem Schmerz.

2. Verwendung gemäß Anspruch 1, **dadurch gekennzeichnet, dass** R eine Arylalkylgruppe ist, worin die Alkyleinheit zwischen ein und vier Kohlenstoffatome hat.

3. Verwendung gemäß Anspruch 1 oder 2, **dadurch gekennzeichnet, dass** R eine Arylalkylgruppe ist, worin die Aryleinheit ein Phenyl- oder Naphthylring ist.

4. Verwendung gemäß Anspruch 1, **dadurch gekennzeichnet, dass** R eine n-Butylgruppe ist.

5. Verwendung gemäß Anspruch 1, **dadurch gekennzeichnet, dass** R eine Cyclohexylgruppe ist.

## Revendications

1. Utilisation d'un composé de formule 1 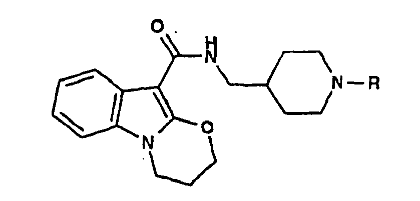 dans laquelle
R est H, une chaîne alkyle linéaire ou ramifiée de 1 à 12 atomes de carbone ou un groupe cyclohexyle ou arylalkyle,
et de ses sels d'addition d'acides avec des acides organiques ou inorganiques pharmaceutiquement acceptables,
pour la préparation d'une composition pharmaceutique destinée au traitement des douleurs neuropathiques.

2. Utilisation selon la revendication 1, **caractérisée en ce que** R est un groupe arylalkyle dans lequel la partie alkyle a de 1 à 4 atomes de carbone.

3. Utilisation selon la revendication 1 ou 2, **caractérisée en ce que** R est un groupe arylalkyle dans lequel la partie aryle est un cycle phényle ou naphtyle.

4. Utilisation selon la revendication 1, **caractérisée en ce que** R est un groupe n-butyle.

5. Utilisation selon la revendication 1, **caractérisée en ce que** R est un groupe cyclohexyle.
